# EUROPEAN PATENT APPLICATION

(11) **EP 4 082 561 A1**
(43) Date of publication of application: **02.11.2022**
(21) Application number: 20904974.1
(22) Date of filing: 24.12.2020
(51) Int. Cl.: A61K 38/26, A61K 47/60, A61K 47/68, A61P 19/10, A61P 19/02, C07K 14/605

(54) **PHARMACEUTICAL COMPOSITION FOR PREVENTING OR TREATING METABOLIC BONE DISEASES, COMPRISING GLP-2 OR CONJUGATE THEREOF**

(30) Priority: 24.12.2019 KR 20190174117
(71) Applicant: Hanmi Pharm. Co., Ltd., Hwaseong-si, Gyeonggi-do 18536 (KR)
(72) Inventor: CHOI, Jae Hyuk, Hwaseong-si, Gyeonggi-do 18469 (KR); LEE, Jin Bong, Hwaseong-si, Gyeonggi-do 18469 (KR); LEE, Sang Hyun, Hwaseong-si, Gyeonggi-do 18469 (KR); LEE, Sang Don, Hwaseong-si, Gyeonggi-do 18469 (KR); KIM, Min Young, Hwaseong-si, Gyeonggi-do 18469 (KR)
(74) Representative: Mewburn Ellis LLP
(86) International application number: PCT/KR2020/019069
(87) International publication number: WO 2021/133087

(57) **Abstract**

The present invention relates to the use of GLP-2 or a long-acting conjugate thereof for preventing or treating metabolic bone diseases.

## Description

### [Technical Field]

The present invention relates to the use of GLP-2 or a long-acting conjugate thereof for preventing or treating metabolic bone diseases.

### [Background Art]

Glucagon-like peptide-2 (GLP-2) is a 33-amino-acid peptide hormone which is produced by the intestinal endocrine L cell upon nutrient ingestion. GLP-2 stimulates mucosal growth in the small and large intestines and suppresses apoptosis and promotes growth of intestinal cells and crypt cells. Furthermore, GLP-2 enhances absorption of nutrients in the small intestine and reduces intestinal permeability. In addition, GLP-2 suppresses gastric emptying and gastric acid secretion, while increasing an intestinal blood flow rate and relaxing intestinal smooth muscle.

Metabolic bone disease (MBD) is a broad term that refers to bone deformities caused by various types of disorders, and is caused by an imbalance of calcium, phosphorus, magnesium, vitamin D, *etc*. In particular, calcium deficiency is known to be the main cause. Examples of the metabolic bone disease include osteoporosis, osteomalacia, rickets, cystic fibrous osteomyelitis, Paget's disease, *etc.*

Meanwhile, osteoporosis, a representative example of metabolic bone disease, is an unavoidable symptom for the elderly, particularly postmenopausal women, although there is a difference in degree, and as the population ages in developed countries, interest in osteoporosis and its therapeutic agents is gradually increasing. Osteoporosis refers to a state in which the amount of bone is significantly reduced compared to that of normal people, and is a common metabolic disease that weakens the ability to withstand weight or mechanical pressure and causes bones to fracture easily even with a slight impact such as a light fall indoors.

Bone in our body repeats the process of being resorbed and reproduced, and it is believed that metabolic bone disease is caused by the imbalance between bone formation and bone resorption. It is known that promoting bone formation and inhibiting bone degradation is an important therapeutic mechanism for treating metabolic bone diseases including osteoporosis. Accordingly, drugs are being developed for maintaining the current bone mass by increasing bone formation or preventing bone loss. Bisphosphate, calcitonin, or estrogen, *etc.* are used as drugs for inhibiting bone resorption, and parathyroid hormone is used to increase bone formation (Korea Patent No. 10-1964376).

Until now, the development of a therapeutic agent for metabolic bone diseases using glucagon-like peptide-2 has been incomplete, and there is a need for continuous therapeutic development.

### [Disclosure]

### [Technical Problem]

It is one object of the present invention to provide a pharmaceutical composition for preventing or treating metabolic bone diseases, including glucagon-like peptide-2 (GLP-2).

It is another object of the present invention to provide a method for preventing or treating metabolic bone diseases, including: administering GLP-2 or a composition containing the same into an individual in need thereof.

It is still another object of the present invention to provide the use of GLP-2 or a composition containing the same in the production of medicament for preventing or treating metabolic bone diseases.

### [Technical Solution]

One aspect for implementing the present invention provides a composition for preventing, treating, or improving metabolic bone diseases, including glucagon-like peptide-2 (GLP-2).

In one embodiment, the present invention relates to a pharmaceutical composition for preventing, treating, or improving metabolic bone diseases, including GLP-2.

In another embodiment, the present invention relates to a food composition for preventing or improving metabolic bone diseases, including GLP-2.

As the composition according to the preceding embodiments, the present invention relates to a pharmaceutical composition for preventing or treating metabolic bone diseases, comprising: a pharmaceutically acceptable excipient; and glucagon-like peptide-2 (GLP-2) in in a pharmaceutically effective amount.

As the composition according to the preceding embodiments, the GLP-2 is characterized in that it is native GLP-2; or a GLP-2 derivative in which a variation selected from the group consisting of substitution, addition, deletion, modification and a combination thereof has occurred in one or more amino acids in a native GLP-2 sequence.

As the composition according to the preceding embodiments, the GLP-2 derivative has a variation in at least one amino acid among amino acids at positions 1, 2, 30, and 34 in the amino acid sequence of SEQ ID NO: 1.

As the composition according to the preceding embodiments, the GLP-2 derivative includes an amino acid sequence represented by General Formula 1 below:

[General Formula 1] X₁X₂DGSFSDEMNTILDNLAARDFINWLIQTX₃₀ITDX₃₄ (SEQ ID NO: 9)

wherein,
X₁ is histidine, imidazoacetyldeshistidine, desaminohistidine, β-hydroxyimidazopropionyldeshistidine, *N*-dimethylhistidine, or β-carboxyim idazopropionyldeshistidine;
X₂ is alanine, glycine, or Aib (2-aminoisobutyric acid);
X₃₀ is lysine or arginine; and
X₃₄ is absent, or lysine, arginine, glutamine, histidine, 6-azidolysine, or cysteine.

As the composition according to the preceding embodiments, the GLP-2 derivative has an amino acid sequence, wherein
(1) X₁ is imidazoacetyldeshistidine, X₂ is glycine, X₃₀ is lysine, and X₃₄ is cysteine,
(2) X₁ is imidazoacetyldeshistidine, X₂ is glycine, X₃₀ is lysine, and X₃₄ is lysine,
(3) X₁ is imidazoacetyldeshistidine, X₂ is glycine, X₃₀ is arginine, and X₃₄ is lysine,
(4) X₁ is imidazoacetyldeshistidine, X₂ is glycine, X₃₀ is lysine, and X₃₄ is 6-azidolysine,
(5) X₁ is imidazoacetyldeshistidine, X₂ is glycine, X₃₀ is arginine, and X₃₄ is cysteine,
(6) X₁ is imidazoacetyldeshistidine, X₂ is Aib, X₃₀ is lysine, and X₃₄ is cysteine, or
(7) X₁ is histidine, X₂ is Aib, X₃₀ is lysine, and X₃₄ is cysteine.

As the composition according to the preceding embodiments, the GLP-2 derivative includes an amino acid sequence represented by General Formula 2 below:

[General Formula 2] X₁X₂DGSFSDEMNTILDNLAARDFINWLIQTX₃₀ITDX₃₄ (SEQ ID NO: 10)

wherein,
X₁ is histidine, imidazoacetyldeshistidine, desaminohistidine, β-hydroxyimidazopropionyldeshistidine, *N*-dimethylhistidine, or β-carboxyimidazopropionyldeshistidine;
X₂ is alanine, glycine, or Aib (2-aminoisobutyric acid);
X₃₀ is lysine or arginine; and
X₃₄ is any one or more amino acids or any one or more amino acids in which variations occur.

As the composition according to the preceding embodiments, the GLP-2 derivative is an amino acid sequence selected from the group consisting of SEQ ID NOS: 2 to 8.

As the composition according to the preceding embodiments, the metabolic bone disease is osteoporosis, osteopenia, arthritis, periodontal disease, osteopsathyrosis, or osteomalacia.

As the composition according to the preceding embodiments, the composition promotes bone formation; inhibits bone degradation; or promotes bone formation and inhibits bone degradation.

As the composition according to the preceding embodiments, the metabolic bone disease is osteoporosis.

As the composition according to the preceding embodiments, the composition has one or more of the following properties upon administration:
(i) an increase in the blood concentration of osteocalcin(osteocalcin/bone Gla-protein, OC/BGP);
(ii) a decrease in the blood concentration of osteoprotegerin (OPG); and
(iii) a decrease in the blood concentration of C-telopeptide of collagen type 1 (CTX-1).

As the composition according to the preceding embodiments, the GLP-2 is unmodified or amidated at the C-terminus thereof.

As the composition according to the preceding embodiments, the GLP-2 is in the form of a long-acting conjugate to which a biocompatible material capable of increasing its *in vivo* half-life is bound.

As the composition according to the preceding embodiments, the conjugate is represented by Chemical Formula 1 below:

[Chemical Formula 1] X-Lₐ-F

wherein,
X is GLP-2 (native GLP-2 or a GLP-2 derivative);
L is a linker containing ethylene glycol repeating units;
a is 0 or a native number with the proviso that when a is 2 or more, each L is independent of the other;
F is an immunoglobulin Fc region; and
the "-" represents a covalent bond.

As the composition according to the preceding embodiments, the F is an immunoglobulin Fc region.

As the composition according to the preceding embodiments, the immunoglobulin Fc region is aglycosylated.

As the composition according to the preceding embodiments, the immunoglobulin Fc region is an IgG4 Fc region.

As the composition according to the preceding embodiments, the immunoglobulin Fc region is an aglycosylated Fc region derived from human IgG4.

As the composition according to the preceding embodiments, the F is a dimer consisting of two polypeptide chains, and one end of L is linked to only one of the two polypeptide chains.

As the composition according to the preceding embodiments, the L is polyethylene glycol.

As the composition according to the preceding embodiments, the formula weight of the ethylene glycol repeating unit moiety in L is in the range of 1 kDa to 100 kDa.

Another aspect of the present invention provides a method for preventing or treating metabolic bone diseases, including: administering GLP-2, a GLP-2 long-acting conjugate or a composition containing the same into an individual in need thereof.

Still another aspect of the present invention provides the use of GLP-2 or a composition containing the same for preventing or treating metabolic bone diseases.

Yet another aspect of the present invention provides the use of GLP-2 or a composition containing the same in the production of medicament for preventing or treating metabolic bone diseases.

### [Advantageous Effects]

The composition including GLP-2 according to the present invention can be applied to the treatment of bone-related diseases including osteoporosis.

### [Brief Description of Drawing]

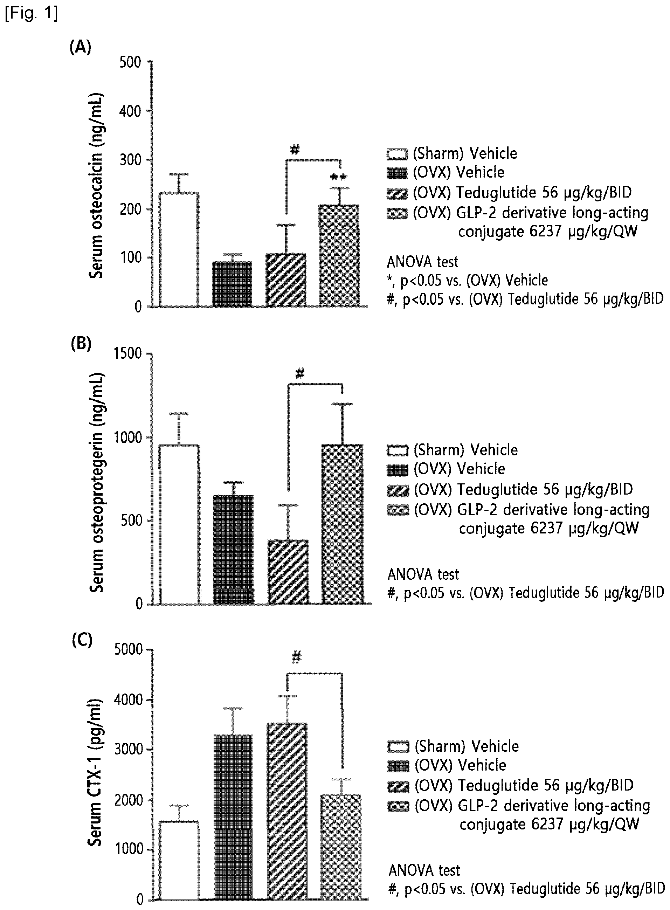

Fig. 1 a diagram confirming the therapeutic effect of the GLP-2 long-acting conjugate for metabolic bone disease by changing the blood concentrations of osteocalcin (OC/BGP), osteoprotezerin (OPG), and C-telopeptide of collagen type 1 (CTX-1).

### [Detailed Description of Preferred Embodiments]

The present application will be described in detail as follows.

Meanwhile, each description and embodiment disclosed herein can be applied to other descriptions and embodiments, respectively. That is, all combinations of various elements disclosed herein fall within the scope of the present application. Further, the scope of the present application is not limited by the specific description described below.

Additionally, those of ordinary skill in the art may be able to recognize or confirm, using only conventional experimentation, many equivalents to the particular aspects of the invention described herein. Furthermore, it is also intended that these equivalents be included in the present application.

Over the entire specification of the present invention, the conventional one-letter and three-letter codes for amino acids are used. Additionally, the amino acids mentioned herein are abbreviated according to the nomenclature rules of the IUPAC-IUB as follows:

| | | | |
|---|---|---|---|
| alanine | A | arginine | R |
| asparagine | N | aspartic acid | D |
| cysteine | C | glutamic acid | E |
| glutamine | Q | glycine | G |
| histidine | H | isoleucine | I |
| leucine | L | lysine | K |
| methionine | M | phenylalanine | F |
| proline | P | serine | S |
| threonine | T | tryptophan | W |
| tyrosine | Y | valine | V |

One aspect for implementing the present invention provides a pharmaceutical composition for preventing or treating bone diseases, including glucagon-like peptide-2 (GLP-2). Specifically, the bone disease may be a metabolic bone disease, more specifically a bone degenerative disease, but is not particularly limited thereto.

As one specific example, the present invention provides a pharmaceutical composition for preventing or treating metabolic bone diseases, including: a pharmaceutically acceptable excipient; and glucagon-like peptide-2 (GLP-2) in a pharmaceutically effective amount.

As used herein, the term "glucagon-like peptide-2" or GLP-2 refers to a substance in the form of a polypeptide, or a conjugate in which a non-polypeptide moiety is linked to a polypeptide as an agonist of human glucagon-like peptide-2 receptor. In the present invention, glucagon-like peptide-2 or GLP-2 includes not only native human GLP-2, but also derivatives thereof, and conjugates thereof. The GLP-2 may be included in a pharmacologically effective amount in the pharmaceutical composition as an active ingredient included in the pharmaceutical composition of the present invention.

The amino acid sequence of the native GLP-2 is as follows:
GLP-2(1-33)
HADGSFSDEMNTILDNLAARDFINWLIQTKITD (SEQ ID NO: 1)

In the present invention, the "GLP-2 receptor agonist" refers to a substance that binds to *in vivo* or isolated human glucagon-like peptide-2 (GLP-2) receptor and produces the same or similar physiological activity as native GLP-2. For example, a GLP-2 agonist may include native GLP-2 or a GLP-2 derivative.

As used herein, the "GLP-2 derivative" may include a peptide having one or more differences in amino acid sequence compared to native GLP-2; a peptide in which the native GLP-2 sequence is modified through modification; and/or mimics of native GLP-2 having a function of preventing, treating, and/or improving metabolic bone diseases, such as native GLP-2. Specifically, the GLP-2 derivative may be those in which a variation selected from the group consisting of substitution, addition, deletion, modification and a combination thereof has occurred in one or more amino acids in the native GLP-2 sequences, but is not limited thereto.

In one specific embodiment, the GLP-2 of the present invention may have the N-terminal amino group substituted, removed, or modified, but is not limited thereto. In order to prevent binding to the N-terminus, which is an important site for the *in vivo* activity of GLP-2, the GLP-2 of the present invention may be prepared by a method of removing the alpha amino group of the N-terminal histidine, a method of synthesizing the N-terminal amino group by substituting it with a hydroxyl group or a carboxyl group, a method of removing the alpha carbon of the N-terminal histidine and the N-terminal amino group bonded to the alpha carbon to leave only an imidazo-acetyl functional group, and a method of modifying the N-terminal amino group with two methyl groups, *etc.,* when preparing a long-acting conjugate.

Specifically, the GLP-2 derivative may be imidazoacetyl-deshistidyl-GLP-2 (CA-GLP-2) in which the N-terminal amino group bonded to the alpha carbon and the alpha carbon of the histidine residue, which is the first N-terminal amino acid of GLP-2 has been removed; desaminohistidyl GLP-2 (DA-GLP-2) from which the N-terminal amino group of GLP-2 has been removed; β-hydroxyimidazopropionyldeshistidyl GLP-2 (HY-GLP-2) in which the N-terminal amino group of GLP-2 is substituted with a hydroxyl group; N-dimethylhistidyl GLP-2 (DM-GLP-2) in which the N-terminal amino group of GLP-2 is modified with two methyl groups; or β-carboxyimidazopropionyl-deshistidyl-GLP-2 (CX-GLP-2) in which the N-terminal amino group of GLP-2 is substituted with a carboxyl, but is not limited thereto. In one non-limiting example, the material structure used to prepare the GLP-2 derivative is as follows:

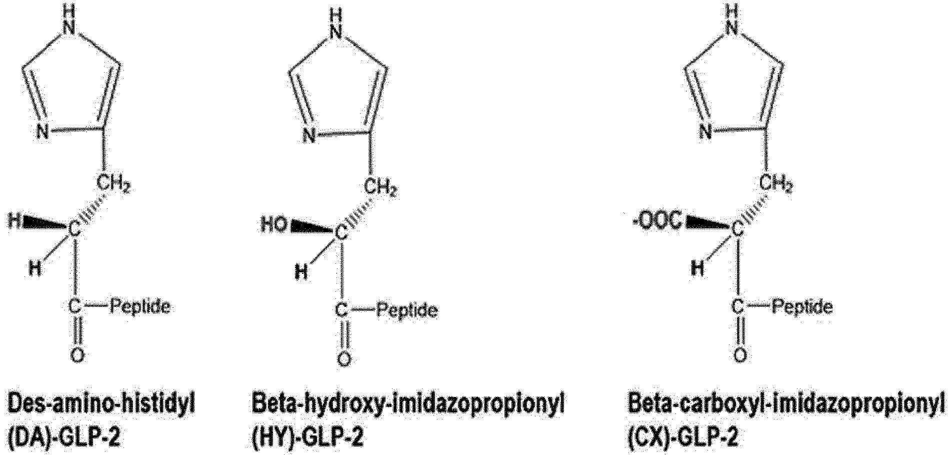

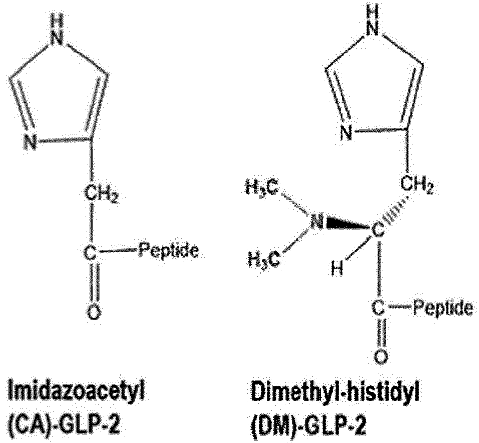

In one specific embodiment, the GLP-2 derivative may have a variation in at least one amino acid among amino acids at positions 1, 2, 30, and 34 in the amino acid sequence of SEQ ID NO: 1.

Specifically, the variation may be a variation selected from the group consisting of substitution, addition, deletion, modification, and combinations thereof of at least one amino acid, wherein the added amino acid may be a non-natural amino acid (e.g., D-amino acid), and substitution of non-natural amino acids other than natural amino acids is also possible. The added amino acid sequence may be derived from native GLP-2, but is not limited thereto.

Specifically, the GLP-2 derivative of the present invention may have an identity of 80%, 85%, 90%, 95%, 96%, 97%, 98%, 99%, or more to the native GLP-2 in the amino acid sequence, or may be in the form in which part of an amino acid residue of GLP-2 is chemically substituted (e.g., alpha-methylation, alpha-hydroxylation), removed (e.g., deamination) or modified (e.g., N-methylation), but is not limited thereto.

In one specific embodiment, the GLP-2 derivative may include an amino acid sequence of General Formula below, but is not limited thereto:

[General Formula 1] X₁X₂DGSFSDEMNTILDNLAARDFINWLIQTX₃₀ITDX₃₄ (SEQ ID NO: 9)

wherein,
X₁ is histidine, imidazoacetyldeshistidine, desaminohistidine, β-hydroxyim idazopropionyldeshistidine, *N*-dimethylhistidine, or β-carboxyimidazopropionyldeshistidine;
X₂ is alanine, glycine, or Aib (2-aminoisobutyric acid);
X₃₀ is lysine or arginine; and
X₃₄ is absent, or lysine, arginine, glutamine, histidine, 6-azidolysine, or cysteine.

In another specific embodiment, the GLP-2 derivative may include an amino acid sequence of General Formula 2 below, but is not limited thereto:

[General Formula 2] X₁X₂DGSFSDEMNTILDNLAARDFINWLIQTX₃₀ITDX₃₄ (SEQ ID NO: 10)

wherein,
X₁ is histidine, imidazoacetyldeshistidine, desaminohistidine, β-hydroxyimidazopropionyldeshistidine, *N*-dimethylhistidine, or β-carboxyimidazopropionyldeshistidine;
X₂ is alanine, glycine, or Aib (2-aminoisobutyric acid);
X₃₀ is lysine or arginine; and
X₃₄ is any one or more amino acids or any one or more amino acids in which variations occur.

Specifically, among the amino acid sequences of General Formula 1 or 2, the same sequence as SEQ ID NO: 1 may be excluded from the GLP-2 derivative, but is not limited thereto.

Specifically, the GLP-2 derivative of the present invention may have a substitution of alanine at the 2nd amino acid of native GLP-2 with glycine or Aib (2-aminoisobutyric acid), a substitution of lysine at the 30th amino acid of native GLP-2 with arginine, or a combination thereof, but is not limited thereto. Additionally, the GLP-2 derivative may be introduced with a thiol group (*e.g*., cysteine), an amino group (*e.g*., lysine, arginine, glutamine or histidine), or an azide group (*e.g*., 6-azidolysine) to the C-terminus (*e.g*., 33rd amino acid), but is not limited thereto.

Since the conjugation occurs in the introduced group when preparing the long-acting conjugate of GLP-2 derivative, these may be used to prepare a GLP-2 conjugate, the binding site of which is selectively controlled. Specifically, the hydroxyl group, thiol group, amino group, or azide group of the GLP-2 derivative may be conjugated to one end of a non-peptidyl linker, and a material (*e.g*., an immunoglobulin Fc region) capable of increasing the *in vivo* half-life may be conjugated to the other end of the non-peptidyl linker, but these are not limited thereto. The thiol group, amino group, or azide group may be introduced by adding an amino acid to GLP-2, but is not limited thereto. The thiol group may be introduced by adding cysteine (C) to GLP-2; the amino group may be introduced by adding lysine (K), arginine (R), glutamine (Q), or histidine (H) to GLP-2; and the azide group may be introduced by adding 6-azido-lysine (_{Az}K) to GLP-2, but these are not limited thereto.

Specifically, in the GLP-2 derivative, at least one of the residues may be cysteine, lysine, arginine, glutamine, histidine, or 6-azido-lysine, but is not limited thereto.

Specifically, the GLP-2 derivative of the present invention may include the substitution of alanine, which is the 2nd amino acid of native GLP-2, with glycine and the introduction of a thiol group (for example, cysteine) into the C-terminus of the GLP-2; and more specifically, the GLP-2 derivative may comprise imidazoacetyldeshistidine in which an α-carbon of a histidine residue, which is the first amino acid at the N-terminus of the GLP-2, and the N-terminal amino group bound to the α-carbon are removed (for example, it may have an amino acid sequence of SEQ ID NO: 2), but is not limited thereto.

Specifically, the GLP-2 derivative of the present invention may include the substitution of alanine, which is the 2nd amino acid of native GLP-2, with glycine, and the introduction of an amino group (*e.g*., lysine) into the C-terminus; and more specifically, the GLP-2 derivative may include imidazoacetyldeshistidine in which an α-carbon of a histidine residue, which is the first amino acid at the N-terminus of the GLP-2, and the N-terminal amino group bound to the α-carbon are removed (for example, it may have an amino acid sequence of SEQ ID NO: 3), but is not limited thereto.

Specifically, the GLP-2 derivative of the present invention may include the substitution of alanine, which is the 2nd amino acid of native GLP-2, with glycine, the substitution of lysine, which is the 30th amino acid of native GLP-2, with arginine, and the introduction of an amino group (*e.g*., lysine) into the C-terminus; and more specifically, the GLP-2 derivative may include imidazoacetyldeshistidine in which an α-carbon of a histidine residue, which is the first amino acid at the N-terminus of the GLP-2, and the N-terminal amino group bound to the α-carbon are removed (for example, it may have an amino acid sequence of SEQ ID NO: 4), but is not limited thereto.

Specifically, the GLP-2 derivative of the present invention may include the substitution of alanine, which is the 2nd amino acid of native GLP-2, with glycine, and the introduction of an azide group (*e.g*., 6-azido-lysine) into the C-terminus; and more specifically, the GLP-2 derivative may include imidazoacetyldeshistidine in which an α-carbon of a histidine residue, which is the first amino acid at the N-terminus of the GLP-2, and the N-terminal amino group bound to the α-carbon are removed (for example, it may have an amino acid sequence of SEQ ID NO: 5), but is not limited thereto.

Specifically, the GLP-2 derivative of the present invention may include the substitution of alanine, which is the 2nd amino acid of native GLP-2, with glycine, the substitution of lysine, which is the 30th amino acid of native GLP-2, with arginine, and the introduction of a thiol group (*e.g*., cysteine) into the C-terminus; and more specifically, the GLP-2 derivative may include imidazoacetyldeshistidine in which an α-carbon of a histidine residue, which is the first amino acid at the N-terminus of the GLP-2, and the N-terminal amino group bound to the α-carbon are removed (for example, it may have an amino acid sequence of SEQ ID NO: 6), but is not limited thereto.

Specifically, the GLP-2 derivative of the present invention may include the substitution of alanine, which is the 2^{nd} amino acid of native GLP-2, with 2-aminoisobutyric acid, and the introduction of a thiol group (*e.g*., cysteine) into the C-terminus (for example, it may have an amino acid sequence of SEQ ID NO: 8); and more specifically, the GLP-2 derivative may include imidazoacetyldeshistidine in which an α-carbon of a histidine residue, which is the first amino acid at the N-terminus of the GLP-2, and the N-terminal amino group bound to the α-carbon are removed (for example, it may have an amino acid sequence of SEQ ID NO: 7), but is not limited thereto.

The GLP-2 derivatives of SEQ ID NOS: 2 to 8 are shown in Table 1 below.

**[Table 1]**

| Name | Sequence | SEQ ID NO: |
|---|---|---|
| CA GLP-2 KC | *_{ca}*HGDGSFSDEMNTILDNLAARDFINWLIQTKITDC | 2 |
| CA GLP-2 KK | *_{ca}*HGDGSFSDEMNTILDNLAARDFINWLIQIKIIDK | 3 |
| CA GLP-2 RK | *_{ca}*HGDGSFSDEMNTILDNLAARDFINWLIQTRITDK | 4 |
| CA GLP-2 K_{AZ}K | *_{ca}*HGDGSFSDEMNIYLDMLAARDFINWLIQTKITD_{Az}K | 5 |
| CA GLP-2 RC | *_{ca}*HGDGSFSDEMNTILDNLAARDFINWLIQTRITDC | 6 |
| CA GLP-2 Aib | *_{ca}*HAibDGSFSDEMNTILDNLAARDFINWLIQTKITDC | 7 |
| GLP-2 Aib | HAibDGSFSDEMNTILDNLAARDFINWLIQTKITDC | 8 |

In Table 1 above, *_{ca}*H indicates one that is substituted with imidazoacetyldeshistidine, instead of histidine; Aib indicates 2-aminoisobutyric acid; and _{Az}K indicates 6-azido-L-lysine.

The GLP-2 derivatives according to the present invention may be peptides including the specific sequences above, or may be peptides consisting (essentially) of the specific sequences above, but the GLP-2 derivatives are not limited thereto.

Meanwhile, although described as a peptide or a GLP-2 derivative "consisting of a particular SEQ ID NO" in the present invention, such expression does not exclude a mutation in the peptide or the GLP-2 derivative that can occur by a meaningless sequence addition upstream or downstream of the amino acid sequence of the corresponding SEQ ID NO, or a naturally occurring mutation therein, or a silent mutation therein, as long as the peptide or GLP-2 derivative having such mutation has an activity the same as or corresponding to the peptide or GLP-2 derivative which consists of an amino acid sequence of the corresponding SEQ ID NO. Even when the sequence addition or a mutation is present, it obviously belongs to the scope of the present invention.

Specifically, in General Formula 1 or 2, (1) X₂ may be glycine or Aib, (2) X₃₀ may be lysine or arginine or, or (3) X₂ may be glycine or Aib and X₃₀ may be lysine or arginine, but these are not limited thereto.

Specifically, in General Formula 1 or 2,
(1) X₁ is imidazoacetyldeshistidine, X₂ is glycine, X₃₀ is lysine, and X₃₄ is cysteine,
(2) X₁ is imidazoacetyldeshistidine, X₂ is glycine, X₃₀ is lysine, and X₃₄ is lysine,
(3) X₁ is imidazoacetyldeshistidine, X₂ is glycine, X₃₀ is arginine, and X₃₄ is lysine,
(4) X₁ is imidazoacetyldeshistidine, X₂ is glycine, X₃₀ is lysine, and X₃₄ is 6-azidolysine,
(5) X₁ is imidazoacetyldeshistidine, X₂ is glycine, X₃₀ is arginine, and X₃₄ is cysteine,
(6) X₁ is imidazoacetyldeshistidine, X₂ is Aib, X₃₀ is lysine, and X₃₄ is cysteine, or
(7) X₁ is histidine, X₂ is Aib, X₃₀ is lysine, and X₃₄ is cysteine, but these are not limited thereto.

In the present invention, the modification for preparing agonists, fragments, variants, and derivatives of native GLP-2 may include all of the modifications using L-type or D-type amino acids and/or non-native amino acids; and/or a modification of native sequence or a post-translational modification (*e.g*., methylation, acylation, ubiquitination, intra-molecular covalent bonding, *etc*.).

In the present invention, the GLP-2 or GLP-2 derivatives may be in the form of a variant where the N-terminus and/or C-terminus, *etc.* thereof is chemically modified or protected by organic groups, or amino acids may be added to the terminus of the GLP-2 or GLP-2 derivatives, for its protection from proteases *in vivo* while increasing its stability.

In particular, in the case of a chemically synthesized peptide, its N- and C-termini are electrically charged. Therefore, in order to remove such charge, the N-terminus of the peptide may be acetylated and/or the C-terminus of the peptide may be amidated, but the peptide modification is not particularly limited thereto.

Specifically, in the present invention, the GLP-2 or GLP-2 derivatives may be non-modified or amidated at the C-terminus thereof, but the modification is not limited thereto.

The GLP-2 or GLP-2 derivative of the present invention can be synthesized through a solid phase synthesis method, can be produced by a recombinant method, and can be produced by commercially.

In the present invention, the GLP-2 may be in the form of a long-acting conjugate in which a biocompatible material for increasing the *in vivo* half-life of a native GLP-2 or GLP-2 derivatives is linked to a native GLP-2 or GLP-2 derivatives, but is not limited thereto. The long-acting conjugate can exhibit an enhanced duration of efficacy compared to GLP-2 or a derivative thereof to which a biocompatible material (*e.g*., immunoglobulin Fc region) is not linked. In the present invention, such a conjugate including GLP-2 or a derivative thereof, in which the half-life is increased by binding of a biocompatible material to the GLP-2 or a derivative thereof, is referred to as a "long-acting conjugate", which may be used interchangeably with "conjugate".

Meanwhile, the conjugate may be one that is non-natively occurring.

In addition, in the long-acting conjugate of GLP-2, the linkage between GLP-2 and a biocompatible material (*e.g*., an immunoglobulin Fc region) may be via a physical or chemical bond, a non-covalent bond or a covalent bond, and specifically a covalent bond, but is not limited thereto.

Additionally, in the long-acting conjugate of GLP-2, the method for linking GLP-2 and a biocompatible material (*e.g*., immunoglobulin Fc region) is not particularly limited, but GLP-2 and a biocompatible material (*e.g*., immunoglobulin Fc region) may be linked to each other via a linker.

Further, Korean Patent Application Laid-Open No. 10-2019-0037181 relating to a long-acting conjugate of GLP-2 is incorporated herein by reference.

One specific embodiment, the long-acting conjugate of GLP-2 of the present invention has the following structure of Chemical Formula 1:

[Chemical Formula 1] X-Lₐ-F

wherein,
X is GLP-2 (native GLP-2 or a GLP-2 derivative);
L is a linker containing ethylene glycol repeating units;
a is 0 or a native number with the proviso that when a is 2 or more, each L is independent of the other;
F is an immunoglobulin Fc region; and
the "-" represents a covalent bond.

More specifically, X and L, and L and F may be linked to each other via a covalent bond, and in this case, the conjugate may be a conjugate in which X, L, and F are each linked via a covalent bond in the order of Chemical Formula 1.

Additionally, F may be directly linked to X (*i.e*., a is 0 in Chemical Formula 1) or may be linked via a linker (L).

In the above conjugate, F is a material which can increase the half-life of X, and it corresponds to a constitution of a moiety constituting the conjugate of the present invention.

The F and the X may be bound to each other via a covalent chemical bond or non-covalent chemical bond; or F and X may be bound to each other through L via a covalent chemical bond, a non-covalent chemical bond, or a combination thereof.

The F, which is the material capable of increasing the half-life of X, may be a biocompatible material, for example, one selected from the group consisting of a polymer, fatty acid, cholesterol, albumin and a fragment thereof, an albumin-binding material, a polymer of repeating units of particular amino acid sequences, an antibody, an antibody fragment, an FcRn-binding material, an *in vivo* connective tissue, a nucleotide, fibronectin, transferrin, a saccharide, heparin, and elastin, but is not particularly limited thereto.

The elastin may be human tropoelastin, which is a water-soluble precursor, and may be a certain sequence of tropoelastin or a polymer of certain repeating units, for example, inclusive of all elastin-analogous polypeptides, but is not particularly limited thereto.

Examples of the polymer may be one selected from the group consisting of polyethylene glycol (PEG), polypropylene glycol, an ethylene glycol-propylene glycol copolymer, polyoxyethylated polyol, polyvinyl alcohol, polysaccharide, polyvinyl ethyl ether, a biodegradable polymer, a lipid polymer, chitins, hyaluronic acid, an oligonucleotide, and a combination thereof, and dextran may be included as the polysaccharide, but are not particularly limited thereto.

In the present specification, the polyethylene glycol encompasses all of the forms of homopolymers of ethylene glycol, PEG copolymers, or monomethyl-substituted PEG polymers (mPEG), but is not particularly limited thereto.

Additionally, the biocompatible material may include poly-amino acids such as poly-lysine, poly-aspartic acid, and poly-glutamic acid, but is not limited thereto.

Additionally, the fatty acid may be one having a binding affinity to albumin *in vivo,* but is not particularly limited thereto.

As an example of F, the F may be an FcRn-binding material, and specifically, the FcRn-binding material may be an immunoglobulin Fc region, more specifically an IgG Fc region, even more specifically a non-glycosylated IgG4 Fc region, but is not particularly limited thereto.

In a specific example of the present invention, the F (*e.g*., immunoglobulin Fc region) is a dimer consisting of two polypeptide chains, and may have a structure in which one end of L is linked only to one of the two polypeptide chains, but is not limited thereto.

In one specific embodiment, the long-acting conjugate of the present invention may be one in which GLP-2 or a derivative thereof and an immunoglobulin Fc region are linked, but is not limited thereto.

In the present invention, the term "immunoglobulin Fc fragment" refers to a region which includes the heavy chain constant region 2 (CH2) and/or heavy chain constant region 3 (CH3) portions, excluding the variable regions of immunoglobulin heavy and light chains. The immunoglobulin Fc fragment may be a constitution constituting the moiety of the conjugate of the present invention. Specifically, it corresponds to F in the Chemical Formula 1 above.

In the present invention, an Fc fragment includes not only the native sequence obtained by papain digestion of immunoglobulin, but also a derivative thereof (*e.g*., sequences in which one or more amino acid residues in the native sequence are modified by deletion, insertion, non-conservative or conservative substitution, or a combination thereof), and is thus different from that of the native form.

The F (*e.g*., immunoglobulin Fc fragment) may have a structure in which two polypeptide chains are linked by a disulfide bond, or a structure in which two polypeptide chains are linked through a nitrogen atom in only one of the two chains, but the structure of the F is not limited thereto. The linkage through the nitrogen atom may be linked to the epsilon N atom or the N-terminus amino group of lysine via reductive amination.

The reductive amination reaction refers to a reaction in which an amine group or amino group of a reactant reacts with an aldehyde of another reactant (*i.e*., a functional group capable of reductive amination) to produce an amine, and an amine bond is formed by a reduction reaction thereafter. The reductive amination reaction is a reaction of organic synthesis widely known in the art.

In one embodiment of the GLP-2 long-acting conjugate of the present invention, the long-acting conjugate may be one in which the immunoglobulin Fc region is linked to a linker through a nitrogen atom at its N-terminus.

Such an immunoglobulin Fc fragment may include a hinge region in the heavy chain constant region, but is not limited thereto.

In the present invention, the immunoglobulin Fc fragment may include a specific hinge sequence in the N-terminus.

As used herein, the term "hinge sequence" refers to a region which is located in the heavy chain and forms a dimer of immunoglobulin Fc fragments through an inter-disulfide bond.

In the present invention, the hinge sequence may be a modified sequence in which part of the hinge sequence having the following amino acid sequence is deleted and thus there is only one cysteine residue in the sequence, but is not limited thereto:
Glu-Ser-Lys-Tyr-Gly-Pro-Pro-Cys-Pro-Ser-Cys-Pro (SEQ ID NO: 11).

The hinge sequence may be one in which the 8th or 11th cysteine residue in the hinge sequence of SEQ ID NO: 11 is deleted and thus only one cysteine residue is included in the sequence. The hinge sequence of the present invention may consist of 3 to 12 amino acids, including only one cysteine residue, but the hinge sequence is not limited thereto. More specifically, the hinge sequence of the present invention may have the following sequences:

Glu–Ser–Lys–Tyr–Gly–Pro–Pro–Pro–Ser–Cys–Pro (SEQ ID NO: 12), Glu–
Ser–Lys–Tyr–Gly–Pro–Pro–Cys–Pro–Ser–Pro (SEQ ID NO: 13), Glu–Ser–Lys–Tyr–
Gly–Pro–Pro–Cys–Pro–Ser (SEQ ID NO: 14), Glu–Ser–Lys–Tyr–Gly–Pro–Pro–Cys–
Pro–Pro (SEQ ID NO: 15), Lys–Tyr–Gly–Pro–Pro–Cys–Pro–Ser (SEQ ID NO: 16),
Glu–Ser–Lys–Tyr–Gly–Pro–Pro–Cys (SEQ ID NO: 17), Glu–Lys–Tyr–Gly–Pro–Pro–
Cys (SEQ ID NO: 18), Glu–Ser–Pro–Ser–Cys–Pro (SEQ ID NO: 19), Glu–Pro–Ser–
Cys–Pro (SEQ ID NO: 20), Pro–Ser–Cys–Pro (SEQ ID NO: 21), Glu–Ser–Lys–Tyr–
Gly–Pro–Pro–Ser–Cys–Pro (SEQ ID NO: 22), Lys–Tyr–Gly–Pro–Pro–Pro–Ser–Cys–
Pro (SEQ ID NO: 23), Glu–Ser–Lys–Tyr–Gly–Pro–Ser–Cys–Pro (SEQ ID NO: 24),
Glu–Ser–Lys–Tyr–Gly–Pro–Pro–Cys (SEQ ID NO: 25), Lys–Tyr–Gly–Pro–Pro–Cys–
Pro (SEQ ID NO: 26), Glu–Ser–Lys–Pro–Ser–Cys–Pro (SEQ ID NO: 27), Glu–Ser–
Pro–Ser–Cys–Pro (SEQ ID NO: 28), Glu–Pro–Ser–Cys (SEQ ID NO: 29), Ser–Cys–
Pro (SEQ ID NO: 30).

More specifically, the hinge sequence may be one which includes the amino acid sequence of SEQ ID NO: 21 (Pro-Ser-Cys-Pro) or SEQ ID NO: 30 (Ser-Cys-Pro), but the hinge sequence is not limited thereto.

In a more specific embodiment of the GLP-2 long-acting conjugate of the present invention, the N-terminal of the immunoglobulin Fc region in the conjugate is proline, and in this conjugate, the Fc region is linked to a linker through the nitrogen atom of the proline.

In a more specific embodiment of the GLP-2 long-acting conjugate of the present invention, the immunoglobulin Fc fragment of the present invention may be in the form of a dimer in which two molecules of the immunoglobulin Fc chain form a homodimer or heterodimer due to the presence of a hinge sequence therein, and in addition, the conjugate of Formula 1 of the present invention may be in the form in which one end of the linker is linked to one chain of the dimeric immunoglobulin Fc fragments, but the immunoglobulin Fc fragment and the conjugate of Formula 1 are not limited thereto.

As used herein, the term "N-terminus" refers to the amino terminus of a protein or polypeptide, and it may include 1, 2, 3, 4, 5, 6, 7, 8, 9, or 10 or more amino acids from the most terminal end or the terminal end of the amino terminus. The immunoglobulin Fc fragment of the present invention may include a hinge sequence in the N-terminus, but the immunoglobulin Fc fragment is not limited thereto.

In addition, the immunoglobulin Fc fragment of the present invention may be an extended Fc fragment, which includes all or part of the heavy chain constant region 1 (CH1) and/or light chain constant region 1 (CL1) excluding the heavy chain and light chain variable regions of an immunoglobulin, as long as it has substantially the same or an improved effect compared to its native type. In addition, the immunoglobulin Fc fragment of the present invention may be a fragment in which some fairly long amino acid sequences corresponding to CH2 and/or CH3 are removed.

For example, the immunoglobulin Fc fragment of the present invention may be 1) a CH1 domain, a CH2 domain, a CH3 domain, and a CH4 domain; 2) a CH1 domain and a CH2 domain; 3) a CH1 domain and a CH3 domain; 4) a CH2 domain and a CH3 domain; 5) a combination between one or two or more domains among a CH1 domain, a CH2 domain, a CH3 domain, and a CH4 domain and an immunoglobulin hinge region (or part of the hinge region); and 6) a dimer of each domain of the heavy chain constant region and a light chain constant region, but the immunoglobulin Fc fragment is not limited thereto.

In addition, in one embodiment of the GLP-2 long-acting conjugate of the present invention, the immunoglobulin Fc region F is a dimer (dimer) consisting of two polypeptide chains, wherein the Fc region dimers F and X are covalently linked through one and the same linker L containing an ethylene glycol repeating unit. In one specific embodiment, X is covalently linked to only one of the two polypeptide chains of the Fc region dimer F via a linker L. In a more specific example, only one molecule of X is covalently linked via L to one of the two polypeptide chains of the Fc region dimer F to which X is linked. In the most specific example, F is a homodimer.

Meanwhile, in another embodiment of the GLP-2 long-acting conjugate of the present invention, it is also possible that two molecules of X are symmetrically bound to one Fc fragment in a dimeric form. In particular, the immunoglobulin Fc and X may be linked to each other by a non-peptide linker, but is not limited to the embodiments described above.

In addition, the immunoglobulin Fc fragment of the present invention includes natural amino acid sequences as well as sequence derivatives thereof. The amino acid sequence derivative means that the sequence of the amino acid is different from that of its natural amino acid due to the presence of deletion, addition, conservative or non-conservative substitution, or a combination thereof in one or more amino acid residues in the sequence of the natural amino acid.

For example, amino acid residues at positions 214 to 238, 297 to 299, 318 to 322, or 327 to 331 in IgG Fc, which are known to be important for linkage, may be used as the sites suitable for modification.

Additionally, various types of derivatives are possible, for example, one where the site capable of forming a disulfide bond is removed; one where several N-terminal amino acids from native Fc are removed; one where a methionine residue is added to the N-terminus of native Fc, *etc.* Additionally, complement binding sites (*e.g*., C1q binding sites) or antibody-dependent cell-mediated cytotoxicity (ADCC) sites may be removed to remove the effector function. The techniques for preparing the sequence derivatives of an immunoglobulin Fc fragment are disclosed in International Publication Nos. WO 97/34631, WO 96/32478, *etc.*

Amino acid substitutions in a protein or peptide that do not alter the entire activity of a molecule are well known in the art (H. Neurath, R. L. Hill, The Proteins, Academic Press, New York, 1979). The most common substitutions occur between amino acid residues of Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly. In some cases, amino acids may be modified by phosphorylation, sulfation, acrylation, glycosylation, methylation, farnesylation, acetylation, amidation, *etc.*

Additionally, the Fc derivatives described above may be those which exhibit the same biological activity as the Fc region of the present invention and have increased structural stability of the Fc region against heat, pH, *etc.*

Additionally, such an Fc fragment may be obtained from a native type isolated from humans or animals (*e.g*., cows, goats, pigs, mice, rabbits, hamsters, rats, guinea pigs, *etc.*) or may be recombinants or derivatives thereof obtained from transformed animal cells or microorganisms. In particular, the Fc fragment may be obtained from native Fc by isolating whole immunoglobulins from human or animal organisms and treating them with a protease. Papain treatment of the Fc fragment generates Fab and Fc fragments, and pepsin treatment of the Fc fragment produces pF' c and F(ab)₂ fragments. These fragments may be subjected to size exclusion chromatography to isolate Fc or pF' c. In a more specific embodiment, the Fc fragment may be a recombinant immunoglobulin Fc fragment where a human-derived Fc fragment is obtained from a microorganism.

Additionally, the immunoglobulin Fc fragment may be in the form of native glycan, increased glycans compared to its native type, decreased glycans compared to its native type, or in a deglycosylated form. The increase, decrease, or removal of the immunoglobulin Fc glycans may be achieved by conventional methods such as a chemical method, enzymatic method, and genetic engineering method using a microorganism. In particular, the immunoglobulin Fc fragment where the glycans are removed from the Fc shows a significant decrease in binding affinity for the complement (C1 q) and a decrease or removal of antibody-dependent cytotoxicity or complement-dependent cytotoxicity, and thus it does not induce unnecessary immune responses *in vivo.* In this regard, an immunoglobulin Fc fragment in a deglycosylated or aglycosylated form may be more suitable to meet the original object of the present invention as a drug carrier.

As used herein, the term "deglycosylation" means removal of glycans from an Fc fragment with an enzyme, and the term "aglycosylation" means that an Fc fragment is produced in an unglycosylated form in prokaryotes, and in a more specific embodiment, in *E*. *coli.*

Meanwhile, the immunoglobulin Fc fragment may be derived from humans or animals (e.g., cows, goats, pigs, mice, rabbits, hamsters, rats, guinea pigs, *etc.*), and in a more specific embodiment, it may be derived from humans.

Additionally, the immunoglobulin Fc fragment may be derived from IgG, IgA, IgD, IgE, IgM, or a combination or hybrid thereof. In a more specific embodiment, the immunoglobulin Fc fragment may be derived from IgG or IgM, which are among the most abundant proteins in human blood, and in an even more specific embodiment, it may be derived from IgG, which is known to enhance the half-lives of ligand-binding proteins. In an even yet more specific embodiment, the immunoglobulin Fc fragment may be an IgG4 Fc fragment, and in the most specific embodiment, it may be an aglycosylated Fc fragment derived from a human IgG4, but the immunoglobulin Fc fragment is not limited thereto.

Additionally, in a specific embodiment, the immunoglobulin Fc fragment, being a human IgG4 fragment, may be in the form of a homodimer in which two monomers are linked through an inter-disulfide bond (an inter-chain form) between cysteines, which are the 3th amino acid of each monomer. In particular, each monomer of the homodimer independently has/or can have an internal disulfide bond between the cysteines at positions 35 and 95; and an internal disulfide bond between the cysteines at positions 141 and 199 (*i.e*., two internal disulfide bonds (an intra-chain form)).

With respect to the number of amino acids, each monomer may consist of 221 amino acids, and the amino acids forming the homodimer may consist of a total of 442 amino acids, but the number of amino acids is not limited thereto. Specifically, the immunoglobulin Fc fragment may be one in which two monomers having the amino acid sequence of SEQ ID NO: 31 (consisting of 221 amino acids) form a homodimer through an inter-disulfide bond between cysteines, which are the 3rd amino acid of each monomer, and in which the monomers of the homodimer independently form an internal disulfide bond between the cysteines at positions 35 and 95 and an internal disulfide bond between the cysteines at positions 141 and 199, but the immunoglobulin Fc fragment is not limited thereto.

As used herein, the term "combination" means that polypeptides encoding single-chain immunoglobulin Fc fragments of the same origin are linked to a single-chain polypeptide of a different origin to form a dimer or multimer. That is, it is possible to prepare a dimer or multimer from two or more fragments selected from the group consisting of Fc fragments of IgG Fc, IgA Fc, IgM Fc, IgD Fc, and IgE Fc.

As used herein, the term "hybrid" means that sequences corresponding two or more immunoglobulin Fc fragments of different origins are present in a single chain of an immunoglobulin constant region. In the present invention, various hybrid forms are possible. For example, the hybrid domain may be composed of one to four domains selected from the group consisting of CH1, CH2, CH3, and CH4 of IgG Fc, IgM Fc, IgA Fc, IgE Fc, and IgD Fc, and may further include a hinge region.

Meanwhile, IgG may be divided into the IgG1, IgG2, IgG3, and IgG4 subclasses, and the present invention may include combinations or hybrids thereof. Preferred are the IgG2 and IgG4 subclasses, and most preferred is the Fc region of IgG4, which rarely has effector functions such as complement-dependent cytotoxicity (CDC).

In addition, the above-described conjugate may have an enhanced duration of efficacy compared to native GLP-2 or to X which is not modified with F, and such a conjugate is not only in the above-described form, but also in the form encapsulated in biodegradable nanoparticles, but is not limited thereto.

Meanwhile, in the Chemical Formula 1, the L may be a non-peptide linker, for example, a linker containing an ethylene glycol repeat unit.

In the present invention, the term "non-peptide linker" includes a biocompatible polymer in which two or more repeat units are linked. The repeat units are linked to each other through any covalent bond which is not a peptide bond. The non-peptide linker may be one constitution that constitutes the moieties of the conjugate of the present invention, and it corresponds to L in Formula 1 above.

As the non-peptide linker that can be used in the present invention, any polymer which has a resistance to proteases *in vivo* can be used without limitation. In the present invention, the non-peptide linker can be used interchangeably with a non-peptide polymer.

Although not particularly limited, the non-peptide linker may be a linker containing an ethylene glycol repeat unit (*e.g*., polyethylene glycol), and additionally, those derivatives which are already known in the art and the derivatives that can easily be prepared at the technological level of those skilled in the art are included in the scope of the present invention.

The repeat unit of the non-peptide linker may be an ethylene glycol repeat unit, and specifically, the non-peptide linker may be one which includes a functional group used for the preparation of the conjugate at an end while including an ethylene glycol repeat unit. The long-acting conjugate according to the present invention may be in the form in which X and F are linked through the functional group, but the long-acting conjugate is not limited thereto. In the present invention, the non-peptide linker may include two, or three or more functional groups, and each functional group may be the same as or different from each other, but the non-peptide linker is not limited thereto.

Specifically, the linker may be polyethylene glycol (PEG) represented by Chemical Formula 2 below, but the linker is not limited thereto: 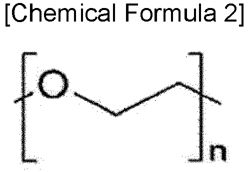 wherein n is 10 to 2,400, n is 10 to 480, or n is 50 to 250, but the range of n is not limited thereto.

In the long-acting conjugate above, the PEG moiety may include not only the -(CH₂CH₂O)ₙ-structure, but also an oxygen atom interposed between a linking element and the -(CH₂CH₂O)ₙ- structure, but the PEG moiety is not limited thereto.

Additionally, in a specific embodiment, the conjugate may have a structure in which an immunoglobulin fragment (F) is linked to GLP-2 or GLP-2 derivatives by a covalent bond through a linker containing an ethylene glycol repeat unit, but the structure of the conjugate is not limited thereto.

The polyethylene glycol is a general term including all of the forms of homopolymers of ethylene glycol, PEG copolymers, and monomethyl-substituted PEG polymers (mPEG), but the polyethylene glycol is not particularly limited thereto.

As the non-peptidyl linker that can be used in the present invention, any non-peptidyl linker may be used without limitation as long as it is a polymer resistant to protease *in vivo,* and specifically, the molecular weight of the non-peptidyl polymer may be in the range of more than 0 kDa to 200 kDa, specifically in the range of 1 kDa to 100 kDa, more specifically in the range of 1 kDa to 50 kDa, further more specifically in the range of 1 kDa to 20 kDa, still further more specifically in the range of 3.4 kDa to 10 kDa, and most specifically about 3.4 kDa, but is not limited thereto.

As used herein, the term "about" refers to a range including all of ±0.5, ±0.4, ±0.3, ±0.2, ±0.1, *etc.,* and it includes all of the values equivalent to those which come immediately after the term "above" or those in a similar range, but is not limited thereto.

Additionally, the non-peptide linker of the present invention, which is linked to the polypeptide corresponding to the F, may include not only a single kind of a polymer but also a combination of different kinds of polymers.

In one specific embodiment, both ends of the non-peptidyl linker may be bound to a thiol group, an amino group, or a hydroxyl group of the immunoglobulin Fc region, and may be bound to a thiol group, an amino group, an azide group, or a hydroxyl group of the GLP-2 derivative.

Specifically, the non-peptidyl linker may include a reactive group capable of binding to each of the immunoglobulin Fc and GLP-2 derivative at both ends. Specifically, the reactive group can bind to a thiol group of cysteine; an amino group located at the N-terminus, lysine, arginine, glutamine, and/or histidine; and/or a hydroxyl group located at the C-terminus of the immunoglobulin Fc region, and can bind to a thiol group of cysteine; an amino group of lysine, arginine, glutamine, and/or histidine; an azide group of azido-lysine; and/or a hydroxyl group of the GLP-2, but the reactive groups are not limited thereto.

More specifically, the reactive group of the non-peptidyl polymer may be one or more selected from the group consisting of an aldehyde group a maleimide group, and a succinimide derivative, but is not limited thereto.

In the above, as an example of the aldehyde group, a propionaldehyde group or butyraldehyde group may be used, but the aldehyde group is not limited thereto.

In the above, as a succinimide derivative, succinimidyl carboxymethyl, succinimidyl valerate, succinimidyl methylbutanoate, succinimidyl methylpropionate, succinimidyl butanoate, succinimidyl propionate, *N*-hydroxysuccinimide, hydroxy succinimidyl, or succinimidyl carbonate may be used, but the succinimide derivative is not limited thereto.

The non-peptidyl linker may be linked to the immunoglobulin Fc and GLP-2 derivative through the reactive groups to be converted to a non-peptidyl polymer linker moiety.

Additionally, the final product produced through reductive amination by an aldehyde bond is much more stable than that linked by an amide bond. The aldehyde reactive group selectively reacts with a N-terminus at a low pH, while it can form a covalent bond with a lysine residue at a high pH (*e.g*., pH 9.0).

The terminal reactive groups of the non-peptidyl polymer of the present invention may be the same as or different from each other. The non-peptidyl polymer may have an aldehyde reactive group at both termini. Alternatively, the non-peptidyl polymer may have an aldehyde group and a maleimide group at each terminus, or may have an aldehyde group and a succinimide reactive group at each terminus, but is not limited thereto.

For example, the non-peptidyl polymer may have a maleimide group at one terminus and an aldehyde group, a propionaldehyde group, or a butyraldehyde group at the other terminus. As another example, the non-peptidyl polymer may have a succinimidyl group at one terminus and a propionaldehyde group or butyraldehyde group at the other terminus.

When a polyethylene glycol having a reactive hydroxy group at both ends thereof is used as the non-peptide polymer, the conjugate of the present invention may be prepared by activating the hydroxy group to various reactive groups by known chemical reactions or by using a commercially available polyethylene glycol having a modified reactive group.

In a specific embodiment, the reactive group of the non-peptide linker may be linked to a cysteine residue of GLP-2 or GLP-2 derivatives, and more specifically, to the -SH group of cysteine, but the non-peptide polymer is not limited thereto.

When maleimide-PEG-aldehyde is used, the maleimide group may be linked to the -SH group of GLP-2 or GLP-2 derivatives by a thioether bond, and the aldehyde group may be linked to the -NH₂ group of the immunoglobulin Fc through reductive alkylation, but is not limited thereto, and this is merely an embodiment.

Through the reductive alkylation, a structure like -PEG-O-CH₂CH₂CH₂NH-immunoglobulin Fc may be formed by linking an amino group at the N-terminus of an immunoglobulin Fc region to an oxygen atom located at one end of the PEG using a linker reactive group having a structure of -CH₂CH₂CH₂-; and a structure in which one end of the PEG is linked to a sulfur atom located at the cysteine of GLP-2 or GLP-2 derivatives through a thioether bond. The thioether bond described above may include the following structure:

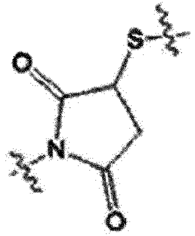

However, the non-peptide polymer is not particularly limited to the above embodiment, but it is merely an embodiment.

Additionally, in the above conjugate, the reactive group of the non-peptide linker may be linked to -NH₂ located at the N-terminus of an immunoglobulin Fc region, but this is merely an embodiment.

In addition, in the conjugate, GLP-2 or a GLP-2 derivative may be linked to a non-peptidyl linker having a reactive group through the C-terminus, but this is merely an embodiment.

As used herein, the term "C-terminus" refers to a carboxy terminus of a peptide, and refers to a position capable of binding with the non-peptidyl polymer for the purpose of the present invention. For example, the C-terminus may include all of the amino acid residue at the most terminal end of the C-terminus and amino acid residues near the C-terminus, and specifically, may include the 1st to 20th amino acid residues from the most terminal end, although the C-terminus is not limited thereto.

Meanwhile, the long-acting conjugate including GLP-2 or a GLP-2 derivatives according to the present invention includes both the peptide itself, a salt thereof (e.g., a pharmaceutically acceptable salt of the peptide), or a solvate thereof.

In addition, the long-acting conjugate may be in any pharmaceutically acceptable form.

The GLP-2 or GLP-2 derivatives of the present invention; a long-acting conjugate including the same; or a composition including the same may have a use for preventing or treating metabolic bone diseases.

Specifically, the composition of the present invention may be used in the prevention or treatment of metabolic bone diseases, and in particular, it may be used without limitation in the prevention or treatment of metabolic bone diseases caused by the imbalance of osteoblasts and osteoclasts. Examples of these diseases include osteoporosis, osteopenia, arthritis, periodontal disease, osteopsathyrosis, or osteomalacia, but are not limited thereto.

The GLP-2 or derivatives thereof according to the present invention; and a long-acting conjugate including the same has an effect of promoting bone formation and/or inhibiting bone degradation by osteoclasts, and thus can be used as an effective therapeutic agent for metabolic bone diseases.

Specifically, the composition of the present invention may exhibit one or more of the following properties upon administration:
(i) an increase in the blood concentration of osteocalcin(osteocalcin/bone Gla-protein, OC/BGP);
(ii) a decrease in the blood concentration of osteoprotegerin (OPG); and
(iii) a decrease in the blood concentration of C-telopeptide of collagen type 1 (CTX-1).

Since the OC/BGP level is a level indicating bone formation, an increase in blood concentration thereof means an effect of promoting bone formation by the long-acting conjugate according to the present invention. Additionally, the OPG and CTX-1 correspond to indicators showing inhibition of osteoclast differentiation and osteoclast activity, and thus, a decrease in blood concentration thereof means an effect of inhibiting bone degradation by the long-acting conjugate according to the present invention. Therefore, based on the above properties, the composition of the present invention can exhibit prevention or treatment effects for metabolic bone diseases by promoting bone formation and/or inhibiting bone degradation.

As used herein, the term "osteoporosis" is a condition in which a calcified bone tissue density is decreased and thus the compact substance of bone is lost gradually, leading to broadening of the marrow cavity. As osteoporosis progresses, bone becomes fragile and consequently bone fractures may readily occur even with a small impact. Bone mass is affected by a variety of factors including genetics, nutrition, hormonal changes, physical exercise and lifestyle habits. Aging, insufficient exercise, being underweight, smoking, low-calcium dietary intake, menopause and ovariectomy are known as pathogenic causes of osteoporosis. Although there is a difference among individuals, it is known that black people exhibit a lower bone resorption level than white people, thus meaning that black people have a higher bone mass. The peak bone mass is generally observed between age 14 and 18, and then the bone mass decreases with aging at a rate of about 1% per year. In particular, bone is continuously decreased from the age of 30 in women and is rapidly reduced due to hormonal changes after menopause. In other words, when reaching the perimenopausal period, a level of estrogen is rapidly decreased. At this time, large numbers of B-lymphocytes are formed as if it happened by interleukin-7 (IL-7), and pre-B cells are accumulated in bone marrow, which consequently leads to an increase in the level of IL-6, thus resulting in an increased activity of osteoclasts and finally a decreased level of bone mass.

In addition, since patients with short bowel syndrome do not receive sufficient nutrients, complications such as osteoporosis, osteopenia, and osteomalacia due to decreased bone density are accompanied.

More specific types of osteoporosis include primary type 1 osteoporosis, primary type 2 osteoporosis, secondary osteoporosis, osteomalacia-like osteoporosis, osteoporosis due to menopause or ovarian resection, and osteoporosis due to short bowel syndrome, but are not particularly limited thereto.

As used herein, the term "osteopenia" refers to a state in which bones are weakened or bone mineral density (BDM) is lower than normal.

As used herein, the term "arthritis" refers to a disease in which inflammatory changes occur in a joint, and includes osteoarthritis, rheumatoid arthritis, *etc.*

As used herein, the term "periodic disease" refers to the inflammatory state of supportive tissue caused by bacteria, and can be divided into gingivitis and periodontitis. The cause of the disease is the formation of dental plaque by oral bacteria due to poor oral hygiene. Dental plaque refers to a mass of bacteria that grows after bacteria adhere to a tooth surface by using a sticky substance in the saliva as an adhesive. If left untreated, the plaque becomes inflamed and sometimes causes bleeding from the gums and bad breath. These symptoms are called gingivitis. As the gingivitis progresses further, the gap between the teeth and the gums becomes deeper, and a paradental cyst develops, and the periodontitis occurs because the bacteria causing the periodontal disease multiply. As periodontitis progresses, even weak stimuli such as brushing may cause bleeding and swelling of the gums, often turning into acute inflammation, causing pain. This inflammation lowers the function of making bones, the bone absorbing effect is increased, the alveolar bone becomes lower, and the alveolar bone is destroyed and eventually teeth are lost. Accordingly, the composition according to the present invention can be usefully used for periodontal disease, particularly alveolar bone loss due to periodontal disease.

As used herein, the term "osteogenesis imperfecta" is a disease called incomplete osteoplasty, and refers to a disease in which bone strength is weak and fractures easily for no particular reason.

As used herein, the term "osteomalacia" refers to temporary swelling and pain in the knee area. The causes are vitamin D deficiency, calcium and phosphate metabolism disorders, and malabsorption of nutrients in the stomach, long-term use of laxatives, insufficient exposure to sunlight, and pregnancy. It refers to a state in which the bone mineral density (BMD) is lower than normal due to the excessive formation of non-calcified osseous tissue in the bone, causing the bone to become soft and to cause movement disorders.

As used herein, the term "prevention" refers to any activity to suppress or delay the onset of metabolic bone diseases by administering GLP-2 or a derivative thereof; or a composition including the same. The term "treatment" refers to all activities that improve or advantageously change the symptoms of metabolic bone diseases by administering GLP-2 or a derivative thereof; or a composition including the same. As used herein, the "prevention or treatment of metabolic bone diseases" includes the prevention and complete or partial treatment of metabolic bone diseases. It also includes any other changes in the patient to reduce, improve the symptoms of metabolic bone diseases, alleviate the pain thereof, reduce in the incidence of metabolic bone diseases, or increase the treatment outcome thereof.

The GLP-2 of the present invention or a derivative thereof; or a long-acting conjugate including the same promotes bone formation and/or inhibits bone degradation to suppress bone loss, thereby maintaining bone mass and bone density, and thus may exhibit a preventive or therapeutic effect on metabolic bone diseases.

As used herein, the term "administration" refers to the introduction of a particular substance into a patient by any appropriate method, and the administration route is not particularly limited, but may be any conventional route that enables delivery of the composition to the target in the body. This may be intraperitoneal administration, intravenous administration, intramuscular administration, subcutaneous administration, intradermal administration, oral administration, local administration, intranasal administration, intrapulmonary administration, intrarectal administration, *etc.*

The pharmaceutical composition of the present invention may further include a pharmaceutically acceptable carrier, excipient or diluent. Such pharmaceutically acceptable carriers, excipients, or diluents may be non-naturally occurring.

As used herein, the term "pharmaceutically acceptable" refers to the properties of having a sufficient amount to exhibit a therapeutic effect and not cause adverse effects, and may be easily determined by those skilled in the art based on factors well known in the medical field, such as the kind of disease, age, weight, health conditions, sex, drug sensitivity of a patient, administration route, administration method, administration frequency, duration of treatment, a drug(s) to be mixed or administered simultaneously, *etc.*

The pharmaceutical composition including GLP-2 of the present invention or a long-acting conjugate thereof may further include a pharmaceutically acceptable carrier. The pharmaceutically acceptable carrier is not particularly limited to, but may include, for oral administration, a binder, a glidant, a disintegrant, an excipient, a solubilizing agent, a dispersant, a stabilizing agent, a suspending agent, a coloring agent, a flavoring agent, *etc*.; for injections, a buffering agent, a preserving agent, an analgesic, a solubilizing agent, an isotonic agent, a stabilizing agent, *etc.,* which may be combined to be used; and for topical administrations, a base, an excipient, a lubricant, a preserving agent, *etc.*

The formulation type of the composition of the present invention may be prepared variously by combining with a pharmaceutically acceptable carrier described above. For example, for oral administration, the composition may be formulated into tablets, troches, capsules, elixirs, suspensions, syrups, wafers, *etc.* For injections, the composition may be formulated into unit-dose ampoules or multi-dose containers. Additionally, the composition may also be formulated into solutions, suspensions, tablets, pills, capsules, sustained-release formulations, *etc.*

Meanwhile, examples of suitable carriers, excipients, and diluents may include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oil, *etc.* Additionally, the composition may further contain a filler, an anti-coagulant, a lubricant, a humectant, a flavoring agent, a preservative, *etc.*

Additionally, the pharmaceutical composition of the present invention may have any one formulation type selected from the group consisting of tablets, pills, powders, granules, capsules, suspensions, liquid medicine for internal use, emulsions, syrups, sterile aqueous solutions, non-aqueous solvents, lyophilized formulations, and suppositories.

Additionally, the composition may be formulated into a preparation of a unit dosage form suitable for the administration into a patient's body, and may specifically be formulated into a preparation useful for administration of protein medicament according to the conventional method in the pharmaceutical field so as to be administered by an oral or parenteral route, including skin, intravenous, intramuscular, intraarterial, intramedullary, intrathecal, intraventricular, pulmonary, transdermal, subcutaneous, intraperitoneal, intranasal, intragastrical, topical, sublingual, vaginal, or rectal route, but the administration routes are not limited thereto.

Additionally, the conjugate may be used by being mixed with various pharmaceutically acceptable carriers such as physiological saline or organic solvents. To increase stability or absorptivity, carbohydrates (*e.g*., glucose, sucrose, or dextran), antioxidants (*e.g*., ascorbic acid or glutathione), chelating agents, low-molecular weight proteins, or other stabilizers, *etc.* may be used as pharmaceutical drugs.

The administration dose and frequency of the pharmaceutical composition of the present invention are determined by the type of drugs (*i.e*., active ingredients), together with various factors, such as the disease to be treated, administration route, patient's age, sex, and body weight, severity of the disease, *etc.*

The total effective amount of the composition of the present invention may be administered to a patient in a single dose or may be administered for a long period of time in multiple doses according to a fractionated treatment protocol. In the pharmaceutical composition of the present invention, the content of active ingredient(s) may vary depending on the severity of the disease. Specifically, the total daily dose of the GLP-2 of the present invention or a long-acting conjugate thereof may be about 0.0001 mg to 500 mg per 1 kg of the body weight of a patient.

However, the effective dose of the GLP-2 or a long-acting conjugate thereof is determined considering various factors including patient's age, body weight, health conditions, sex, disease severity, diet, and excretion rate, as well as administration route and treatment frequency of the pharmaceutical composition. In this respect, those skilled in the art may easily determine the effective dose suitable for a particular use of the pharmaceutical composition of the present invention. The pharmaceutical composition according to the present invention is not particularly limited to the formulation type and administration route and mode, as long as it shows the effects of the present invention.

Including the GLP-2 or GLP-2 derivatives; or a long-acting conjugate including the same in the pharmaceutical composition in a pharmaceutically effective amount refers to a level at which the desired pharmacological activity (*e.g*., prevention, improvement, or treatment of metabolic bone diseases) can be obtained by the GLP-2 or GLP-2 derivatives; or a long-acting conjugate including the same, and in addition, may refer to a level at which toxicities or adverse effects do not occur or occur at an insignificant level in the subject to be administered, or may refer to a pharmaceutically acceptable level, but the level is not limited thereto. The pharmaceutically effective amount as such may be determined by comprehensively considering the number of administration, patient, formulations, *etc.*

Additionally, the pharmaceutical composition of the present invention has excellent *in vivo* duration of efficacy and titer, and thus, the number and frequency of administration of the pharmaceutical preparation of the present invention can be significantly reduced.

Specifically, the pharmaceutical composition of the present invention may be administered once a week, once every two weeks, once every four weeks, or once a month, but the administration is not limited thereto.

Another aspect for implementing the present invention provides a food composition for preventing or treating metabolic bone diseases, including GLP-2 or a long-acting conjugate thereof.

The GLP-2 or a long-acting conjugate thereof, metabolic bone diseases, *etc.,* are as described above.

The food composition may be used as a health functional food. When the composition of the present invention is used as a food additive, the GLP-2 or GLP-2 derivatives; or a long-acting conjugate including the same may be added as it is or used in combination with other foods or food ingredients, and may be appropriately used according to a conventional method. The amount of mixed active ingredients may appropriately be determined depending on the purpose of use (prevention, health, or therapeutic treatment).

As used herein, the term "health functional food" refers to a certain ingredient as a raw material or a food processed by extraction, concentration, purification, mixing of a certain ingredient included in the food raw material for the purpose of health improvement, and refers to a food that is designed and processed to exhibit body modulating function such as body defense, regulation of biorhythm, and prevention and recovery of diseases. The above health functional food composition can perform the functions relating to the prevention and recovery of diseases.

Still another aspect for implementing the present invention provides a method for preventing or treating metabolic bone diseases, including administering the composition including the GLP-2 or a long-acting conjugate thereof into a subject in need thereof.

The GLP-2 or a long-acting conjugate thereof, metabolic bone diseases, *etc.* are as described above.

As used herein, the term "subject" refers to a subject suspected of having metabolic bone, and the subject suspected of having the metabolic bone diseases refers to mammals including humans, rats, cattle, *etc.,* which have or are at risk of developing the metabolic bone diseases, but any subject which can be treated with the conjugate or composition containing the same of the present invention is included without limitation. In a specific example, subjects that benefit from promoting bone formation and/or inhibiting bone degradation are included without limitation.

The method of the present invention may include administering a pharmaceutical composition including GLP-2 or a long-acting conjugate thereof in a pharmaceutically effective amount. An appropriate total daily dose of the pharmaceutical composition may be determined within the scope of correct medical judgment by a practitioner, and the pharmaceutical composition may be administered once or several times in divided doses. However, for the purpose of the present invention, it is preferred that the specific therapeutically effective dose of the pharmaceutical composition for any particular patient be applied differently depending on the kind and degree of responses to be achieved, specific compositions including whether other agents are occasionally used therewith, the patient's age, body weight, health conditions, sex and diet, administration time, administration route, excretion rate of the composition, duration of treatment, other drugs used in combination or simultaneously with the specific compositions, and similar factors well known in the medical field.

Specifically, the preferred total daily dose of the GLP-2 of the present invention or a long-acting conjugate thereof may be about 0.0001 mg to 500 mg per 1 kg of the body weight of a patient, and may be administered once a week, once every two weeks, once every four weeks, or once a month, but dose is not limited thereto.

Yet another aspect for implementing the present invention provides the use of GLP-2 or a long-acting conjugate thereof, or a composition including the same in the prevention or treatment of metabolic bone diseases.

The GLP-2 or a long-acting conjugate thereof, metabolic bone diseases, *etc.* are as described above.

Even another aspect for implementing the present invention provides the use of GLP-2 or a long-acting conjugate thereof, or a composition including the same in the production of medicament for preventing or treating metabolic bone diseases.

The GLP-2 or a long-acting conjugate thereof, metabolic bone diseases, *etc.* are as described above.

Hereinafter, the present application will be described in detail by way of Examples. However, these Examples are given for illustrative purposes only, and are not intended to limit the scope of the invention thereto.

### Example 1: Preparation of Long-Acting Conjugate of GLP-2 Derivatives

For PEGylation of GLP-2 derivative CA GLP-2 RK of SEQ ID NO: 4 of Table 1 to ALD(2) PEG (*i.e*., modified polyethylene glycol having a molecular weight of 3.4 kDa in the ethylene glycol repeating unit moiety, wherein the hydrogens at each terminus are substituted with propionaldehyde groups (3-oxopropyl group); NOF Inc., Japan), which is a modified polyethylene glycol, the reaction was carried out at 2°C to 8°C for 4 to 16 hours with the molar ratio of the GLP-2 derivative and ALD(2) PEG as 1:5 to 1:20 and the concentration of the GLP-2 derivative as 5 mg/mL to 10 mg/mL. Herein, the reaction was carried out in 20 mM HEPES (pH 7.5) and ethanol, and was performed by adding 20 mM sodium cyanoborohydride (NaCNBH₃) as a reducing agent. From the reaction solution, the mono-PEGylated GLP-2 derivative was purified by using a Source 15S column (GE, U.S.A.) using the concentration gradient of a buffer containing sodium citrate (pH 2.0) and ethanol, and potassium chloride.

Thereafter, the reaction was carried out at 2°C to 8°C for 12 to 20 hours, with the molar ratio of the purified mono-PEGylated GLP-2 derivative and the immunoglobulin Fc fragment of SEQ ID NO: 31 as 1:2 to 1:6 and the total protein concentration of 30 mg/mL to 35 mg/mL. Herein, the reaction solution was prepared by adding 20 mM sodium cyanoborohydride as a reducing agent to a 100 mM potassium phosphate buffer (pH 6.0) and isopropanol.

Upon completion of the reaction, the reaction solution was applied to a Source 15Q column (GE, U.S.A.) using the concentration gradient of a bis-Tris buffer (pH 6.5) and sodium chloride, and applied to a Source 15 ISO column (GE, U.S.A.) using the concentration gradient of ammonium sulfate and sodium citrate (pH 5.0 to 5.2) to thereby purify the long-acting conjugate of GLP-2 derivative, which is a conjugate in which the GLP-2 derivative is linked to the immunoglobulin Fc fragment via a linker by a covalent bond.

### Example 2: Changes in the Level of Blood Osteocalcin. Osteoprotegerin. and C-terminal Telopeptide of Collagen Type 1 in Osteoporosis Mice Model According to Administration of GLP-2 Derivative Long-Acting Conjugate

In order to measure the bone protection effect *in vivo* due to the administration of the GLP-2 derivative long-acting conjugate prepared in Example 1, ovariectomized mice (OVX mice, Dae Han Bio Link Co., Ltd.) were used.

Specifically, osteocalcin (OC/BGP) refers to carboxylated osteocalcin that can bind with affinity to hydroxyapatite, a basic mineral component of bone, and when it is present in the blood in large amounts as a bone formation indicator, it means that bone synthesis occurs frequently. Osteoprotegerin (OPG) is an inhibitory indicator of osteoclast differentiation, and it competes with RANKL (receptor activator of nuclear factor *kappa*-B ligand), which plays an important role in osteoclast differentiation, and binds to osteoclast progenitor receptors to hinder the differentiation of osteoclasts. Thus, it is highly present in blood, meaning that bone degradation is inhibited. Since the C-terminal telopeptide (CTX-1) of type 1 collagen is a specific peptide sequence produced by cleavage by osteoclasts during bone resorption, it is proportional to osteoclast activity.

Accordingly, the effect of the GLP-2 derivative long-acting conjugate of the present invention on bone formation and bone degradation was confirmed by confirming the change in the level of blood osteocalcin (OC/BGP), osteoprotegerin (OPG), and C-terminal telopeptide (CTX-1) of type 1 collagen according to the administration of the GLP-2 derivative long-acting conjugate.

First, the ovaries of 7-week-old female mice (C57BL/6J mouse) were removed, and after a one-week recovery period, the mice were divided into 10 mice per group of normal control group (only skin incision and suture without ovariectomy, G1, Sham Vehicle), ovariectomized control group (G2, OVX vehicle), teduglutide administration group after ovariectomy (56 µg/kg/BID) (G3, OVX Teduglutide), and GLP-2 derivative long-acting conjugate administration group after ovariectomy (6237 µg/kg/QW) (G4, OVX GLP-2). (In the present specification, the value of 6237 µg/kg per week expressed as the dose of the GLP-2 derivative long-acting conjugate, 6237 µg is a value excluding the polyethylene glycol linker region from the total mass of the GLP-2 derivative long-acting conjugate used, *i.e*., it is a value expressed based on the sum of the masses of only the polypeptide regions.) After subcutaneous administration of vehicle, teduglutide, or GLP-2 derivative long-acting conjugate to each group for 10 weeks, blood osteocalcin (OC/BGP) levels, osteoprotegerin (OPG) levels and C-telopeptide of type 1 collagen (CTX-1) levels were measured for each group.

As a result of the test, it was confirmed that, in the group administered with the GLP-2 derivative long-acting conjugate (6237 µg/kg/QW), the blood osteocalcin (OC/BGP) level increased as compared to the ovariectomized control group (OVX vehicle) and the teduglutide administration group (56 µg/kg/BID) (Fig. 1 (A)). It was confirmed that in the group administered with the GLP-2 derivative long-acting conjugate (6237 µg/kg/QW), the blood osteoprotegerin (OPG) level showed a tendency to increase as compared to the ovariectomized control group (OVX vehicle), and a significant increase as compared to the teduglutide (56 µg/kg/BID) administration group (Fig. 1 (B)). In addition, it was confirmed that in the group administered with the GLP-2 derivative long-acting conjugate (6237 µg/kg/QW), the level of C-terminal telopeptide (CTX-1) in the blood showed a tendency to decrease compared to the ovariectomized control group (OVX vehicle), and a significant decrease as compared to the teduglutide administration group (56 µg/kg/BID) (Fig. 1(C)).

These results suggest that the administration of the long-acting conjugate of the GLP-2 derivative of the present invention can exhibit a preventive or therapeutic effect on metabolic bone diseases through the mechanism of decreased bone degradation and increased bone formation.

Those of ordinary skill in the art will recognize that the present application may be embodied in other specific forms without departing from its spirit or essential characteristics. The described embodiments are to be considered in all respects only as illustrative and not restrictive. The scope of the present application is therefore indicated by the appended claims rather than by the foregoing description. All changes which come within the meaning and range of equivalency of the claims

## Claims

1. A pharmaceutical composition for preventing or treating metabolic bone diseases, comprising: a pharmaceutically acceptable excipient; and glucagon-like peptide-2 (GLP-2) in a pharmaceutically effective amount.

2. The composition of claim 1, wherein the GLP-2 is a native GLP-2 or a GLP-2 derivative.

3. The composition of claim 2, wherein the GLP-2 derivative has a modification in at least one amino acid among amino acids at positions 1, 2, 30, and 34 in the amino acid sequence of SEQ ID NO: 1.

4. The composition of claim 2, wherein the GLP-2 derivative comprises an amino acid sequence represented by General Formula 1 below:
[General Formula 1] X₁X₂DGSFSDEMNTILDNLAARDFINWLIQTX₃₀ITDX₃₄ (SEQ ID NO: 9)
wherein,
X₁ is histidine, imidazoacetyldeshistidine, desaminohistidine, β-hydroxyimidazopropionyldeshistidine, *N*-dimethylhistidine, or β-carboxyimidazopropionyldeshistidine;
X₂ is alanine, glycine, or Aib (2-aminoisobutyric acid);
X₃₀ is lysine or arginine; and
X₃₄ is absent, or lysine, arginine, glutamine, histidine, 6-azidolysine, or cysteine.

5. The composition of claim 4, wherein the GLP-2 derivative has an amino acid sequence, wherein
(1) X₁ is imidazoacetyldeshistidine, X₂ is glycine, X₃₀ is lysine, and X₃₄ is cysteine,
(2) X₁ is imidazoacetyldeshistidine, X₂ is glycine, X₃₀ is lysine, and X₃₄ is lysine,
(3) X₁ is imidazoacetyldeshistidine, X₂ is glycine, X₃₀ is arginine, and X₃₄ is lysine,
(4) X₁ is imidazoacetyldeshistidine, X₂ is glycine, X₃₀ is lysine, and X₃₄ is 6-azidolysine,
(5) X₁ is imidazoacetyldeshistidine, X₂ is glycine, X₃₀ is arginine, and X₃₄ is cysteine,
(6) X₁ is imidazoacetyldeshistidine, X₂ is Aib, X₃₀ is lysine, and X₃₄ is cysteine, or
(7) X₁ is histidine, X₂ is Aib, X₃₀ is lysine, and X₃₄ is cysteine.

6. The composition of claim 2, wherein the GLP-2 derivative comprises an amino acid sequence represented by General Formula 2 below:
[General Formula 2] X₁X₂DGSFSDEMNTILDNLAARDFINWLIQTX₃₀ITDX₃₄ (SEQ ID NO: 10)
wherein,
X₁ is histidine, imidazoacetyldeshistidine, desaminohistidine, β-hydroxyimidazopropionyldeshistidine, *N*-dimethylhistidine, or β-carboxyimidazopropionyldeshistidine;
X₂ is alanine, glycine, or Aib (2-aminoisobutyric acid);
X₃₀ is lysine or arginine; and
X₃₄ is any one or more amino acids or any one or more amino acids in which modifications occur.

7. The composition of claim 2, wherein the GLP-2 derivative is an amino acid sequence selected from the group consisting of SEQ ID NOS: 2 to 8.

8. The composition of claim 1 or 2, the metabolic bone disease is osteoporosis, osteopenia, arthritis, periodontal disease, osteopsathyrosis, or osteomalacia.

9. The composition of claim 1 or 2, the composition promotes bone formation; inhibits bone degradation; or promotes bone formation and inhibits bone degradation.

10. The composition of claim 9, wherein the metabolic bone disease is osteoporosis.

11. The composition of claim 10, wherein the composition has one or more of the following properties upon administration:
(i) an increase in the blood concentration of osteocalcin(osteocalcin/bone Gla-protein, OC/BGP);
(ii) a decrease in the blood concentration of osteoprotegerin (OPG); and
(iii) a decrease in the blood concentration of C-telopeptide of collagen type 1 (CTX-1).

12. The composition of claim 1 or 2, wherein the GLP-2 is unmodified or amidated at the C-terminus thereof.

13. The composition of claim 1 or 2, wherein the GLP-2 is in the form of a long-acting conjugate to which a biocompatible material capable of increasing its *in vivo* half-life is bound.

14. The composition of claim 13, wherein the conjugate is represented by Chemical Formula 1 below:
[Chemical Formula 1] X-Lₐ-F
wherein,
X is GLP-2 (native GLP-2 or a GLP-2 derivative);
L is a linker containing ethylene glycol repeating units;
a is 0 or a native number with the proviso that when a is 2 or more, each L is independent of the other;
F is an immunoglobulin Fc region; and
the "-" represents a covalent bond.

15. The composition of claim 14, wherein the immunoglobulin Fc region is an aglycosylated IgG4 Fc region.

16. The composition of claim 14, wherein the F is a dimer consisting of two polypeptide chains, and one end of L is linked to only one of the two polypeptide chains.

17. The composition of claim 14, wherein the L is polyethylene glycol.

18. The composition of claim 14, wherein the formula weight of the ethylene glycol repeating unit moiety in L is in the range of 1 kDa to 100 kDa.
